# EUROPEAN PATENT SPECIFICATION

(11) **EP 1 789 023 B1**
(45) Date of publication and mention of the grant of the patent: **12.02.2020**
(21) Application number: 05783096.0
(22) Date of filing: 07.09.2005
(51) Int. Cl.: A61K 9/20, B29C 37/00, G02B 5/18

(54) **MARKING ARTICLES**
MARKIEREN VON GEGENSTÄNDEN
MARQUAGE D'ARTICLES

(30) Priority: 09.09.2004 GB 4199907
(43) Date of publication of application: 30.05.2007
(73) Proprietor: NANO-4-U AG, 6060 Sarnen (CH)
(72) Inventor: WALTER, Harald, 8810 Horgen (CH); SCHNIEPER, Marc, 1213 Onex-Genève (CH); STUCK, Alexander, 5430 Wettingen (CH)
(74) Representative: Riebling, Peter
(86) International application number: PCT/IB2005/002874
(87) International publication number: WO 2006/027688

(56) References cited:
- WO-A-01/10464
- WO-A-03/005839
- US-A- 4 668 523

## Description

The invention relates to a method for the marking of articles for authentication, identification or security.

Products or articles are currently protected against counterfeiting predominantly by attaching security labels to the products or to the packaging of the product. Such labels possess, for example, optical variable devices like holograms or colour changing inks, fluorescent dyes, special printing techniques like microprinting and the like. The main disadvantage of such labels is that they can be removed from the product or the packaging and reused or analysed.

Another approach is to mix highly characteristic isotopes, molecules or particles into the products. For example, short fragments of DNA can be used to mark products.
US patent number 6455157 discloses a method for protecting and marking products by using microparticles which are attached or added to the product. Each microparticle has several colour layers forming a code. All these approaches modify the composition of the product which is very critical for pharmaceuticals or food. The modified products need new approvals, especially for pharmaceutical products. Such approvals have to be obtained from bodies such as the Federal Drugs Agency. A further disadvantage is that the marks are not visible and thus a reading device is necessary to enable their detection.

Another way of marking products is to locally destroy or modify their surface by intense radiation, for example using UV or IR lasers that can write data such as numbers, bar codes, and the like, in various substrates. However, the necessary equipment is expensive and the safety requirements for high intensity radiation sources are stringent and the written information can be easily copied. In addition, the speed of writing the data is low, especially compared with the production speed of pharmaceutical pills and tablets. Further the radiation can destroy the active agent or modify the flavour of the product.

WO 01/10464 A discloses a method for producing an article comprising a diffractive microstructure according to the preamble of claim 1.

WO 03/005839 A describes the use of a specific film-forming material in form of a substrate for the formation of a diffractive microstructure. The substrate has a thickness in the range of 5µm to 50µm and is subsequently sealed into a separate edible article, including primarily candies, but also medicines, such as tablets, that incorporate separately-fabricated edible optical elements such as holographic gratings or diffraction patterns. The pattern is applied to the edible article after the manufacturing of the edible article, in an additional manufacturing step.

US 4 668 523 A describes edible holographic elements comprising organic polymers, and describes methods for making these elements. The polymer is dissolved and applied to a diffraction relief mould followed by a drying step. Thus, the diffraction relief is realised in a soft material, and its fabrication involves a drying step. This method is not applicable to very fast manufacturing of pharmaceutical pills.

In the article Edible Holography: the application of holographic technique to food processing. Eric Begleiter. SPIE Vol. 1461, Practical Holography V (1991) a method of producing a pharmaceutical pill is disclosed comprising forming a diffractive microstructure on or in the surface of the pill, which surface is not completely transparent or metallic reflective but is scattering.

The invention provides a method for producing an article, preferably in form of a pharmaceutical pill, as defined in the appended independent claim, to which reference should now be made. Preferred or advantageous features of the invention are set out in dependent sub-claims.

The disclosure may thus provide a pill having a diffractive microstructure formed in or on a surface thereof to enable identification or authentication thereof.

Diffractive microstructures, particularly gratings, illuminated by polychromatic light show characteristic optical effects. The rainbow effect of holographic microstructures is well known and can be macroscopically structured to form logos, brand names, and the like. Such microstructures typically consist of gratings with periods from 500 nanometres up to a few micrometres. Microstructures with periods below 500nm show special colour effects if they are combined with a high index of refraction layer. Such microstructures are generally called 'zero order microstructures' .

The invention provides a method of producing a pill comprising forming a microstructure on or in the surface of the pill.

Applying such microstructures at an interface of products overcomes the problem of removing and reusing the security feature.

The structuring can only be destroyed. As the optical effects are based on interaction of light with structured interfaces between materials with different refractive indices they are applicable to many products. This is not obvious as most products are not transparent or reflective as is the case for substrates of state of the art holograms but absorbent or possess scattering surfaces. For example, black chocolate consists among other things of certain kinds of polysaccharides which are biopolymers with a refractive index of about 1.5 at 589nm. Structuring the surface with a grating having a period between 0.75µm and 3µm produces a rainbow colour effect due to diffraction of light at the interface between air and chocolate. Although chocolate is normally dark coloured and may be black the absorption only modifies the colour effect without destroying it.

The method comprises the step of coating the article at least in an area covering the microstructure with a transparent layer. This additional coating should be at least partially transparent in the visible spectral range and have an index of refraction higher than the structured material. In this case the microstructures are located at an interface of the product. The optical effects are modified but can still be seen. If the refractive index of the coating is higher than that of the structured material and the period between 200 and 600nm a zero order colour effect can be obtained.

The above and other features and advantages of the invention will be apparent from the following description, by way of example, of embodiments of the invention with reference the accompanying drawings, in which:
Figure 1 shows schematically the diffraction of white light at a microstructured surface of a pharmaceutical pill;
Figure 2a is a photograph of a microstructured piece of chocolate;
Figure 2b shows a piece of chocolate placed on a master during the implementation of the microstructure; and
Figure 3 depicts schematically a production method for adding microstructures to the surface of a pharmaceutical pill.

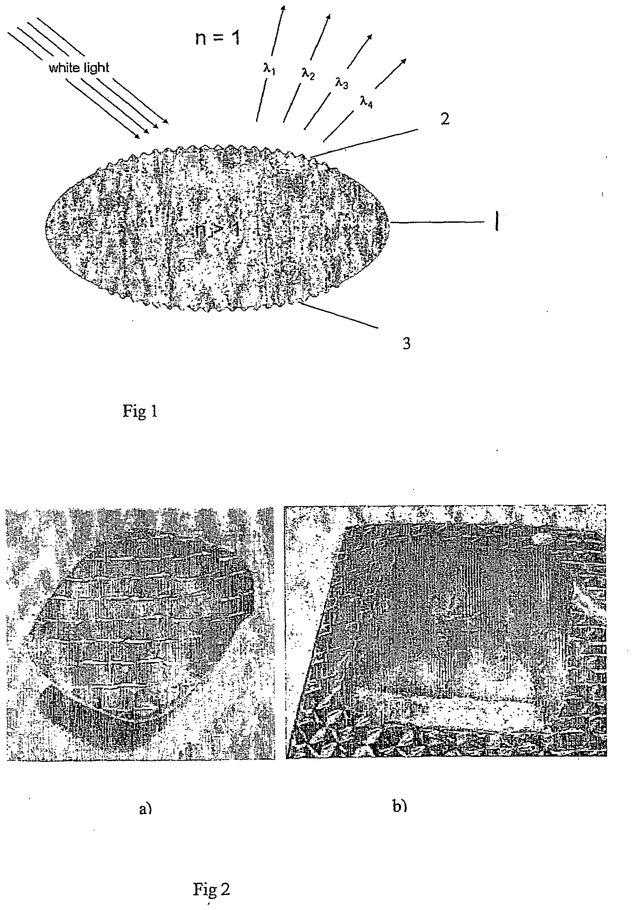

As shown in Figure 1, a pill 1 has a microstructured surface 2 whose structure is shown on an enlarged scale for ease of illustration. In practice, typical periods are between 0.75 microns and 3 microns and the height is around 1 micron. The pills themselves will normally possess a diameter of greater than 3mm. White light incident on the microstructure 2 will be reflected at a number of different wavelengths depending on the angle of incidence of the white light on the microstructure. A further microstructure 3 may be formed on an opposite surface of the pill. This microstructure may have the same properties as the microstructure 2 or may be dimensioned to have different properties.

The production of the microstructure can often be combined with the production of the product. In that case, the cost of adding them can be extremely low. For example, compacting presses may be used for compressing powdered materials into shaped tablets, preforms or briquettes. The structuring of the surface is done by a modified pressing tool which possesses the desired negative microstructure. Similarly, injection moulded products may be formed with the microstructure in place by means of an injection moulding tool which possesses the desired negative microstructure.

Further the microstructures can often only be implemented in a product at a certain step of the production line. For example such products may possess hard or sensitive surfaces at the end of the production process. For such products the invention offers a very high level of security.

Figure 2a illustrates a block of chocolate the surface of which is microstructured and shows diffractive colour effects. Figure 2b shows the piece of chocolate placed on a master during implementation of the microstructure. In this case the microstructuring is achieved by locally melting the surface of the chocolate to emboss the microstructures. The microstructures can be frozen into the surface by cooling the product. While this is illustrated with respect to chocolate it is applicable to any product that can be locally melted and then solidified once the microstructuring has taken place.

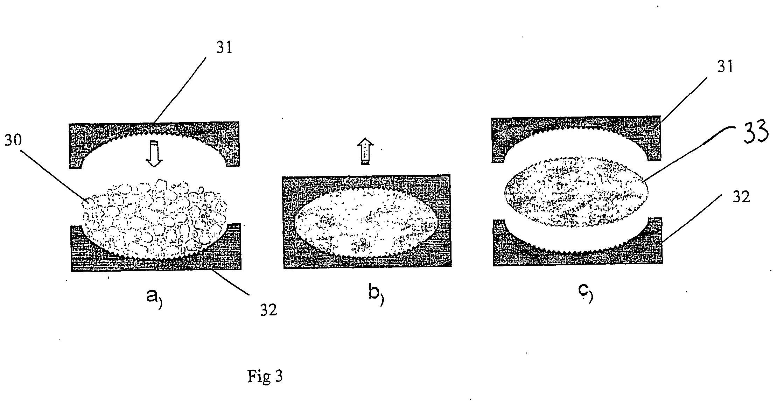

Figure 3 illustrates a possible production method for adding microstructures to the surface of a pharmaceutical pill. First, a raw material in the form of a granular mixture is delivered within one half of a pressing tool 32. The upper half 31 is then lowered onto the lower half 32 compressing the granular mixture 30 between the two halves. This is shown in Figure 3b. The two parts 31 and 32 of the pressing tool are then separated and the pill 33 is ejected. As can be seen from Figure 3c the surface of the pill 33 is formed with the microstructure merely by use of the pressing tool

The invention is applicable to articles of many forms provided that they can be surface treated in some manner to form the microstructure. The invention is particularly useful in protecting articles that are likely to be the subject of counterfeit copies. Such articles might include, for example, spare parts for cars where the original manufacturer wishes to have some means of authenticating spare parts. In this way the customer can ensure that the part being supplied is from the manufacture and not from a counterfeiting source by means of the microstructure which is formed in the surface of the part. The microstructure may be applied during normal materials forming processes such as moulding, pressing, embossing, stamping, etc. Clearly in the case of spare parts the microstructure may be formed on a surface that is not visible once the spare part has been assembled into the product. Thus, for example, body panels for cars could have the microstructure formed on the inside of the panel to avoid any aesthetic problems with the external appearance of the car. A particular application could be in authenticating safety critical parts both for cars and for the aerospace industry.

## Claims

1. A method of producing an article (1) comprising forming a diffractive microstructure (2, 3) at an interface of the article, wherein the method comprises forming the article by means of a pressing or moulding operation, the microstructure (2, 3) being formed in the pressing or moulding process of the article by forming the pressing or moulding tool (31, 32) with a negative version of the microstructure, **characterized in** coating the article at least in an area covering the microstructure with a transparent layer having a higher refractive index than the microstructure.

2. A method as claimed in claim 1 in which the article is a solid pharmaceutical product formed by pressing a powder into a solid mass, the microstructure being formed on a surface of the solid mass during the pressing step by providing a negative master in the pressing tool.

3. A method as claimed in any of claims 1 or 2 in which during the forming step at least the surface of the article is at least locally melted.

4. A method as claimed in any of claims 1 to 3 in which the article or its surface is not completely transparent or metallic reflective but absorbent or scattering.

5. A method as claimed in any of claims 1 to 4, wherein the diffractive microstructure comprises a grating, the grating having a period between 0,75µm and 3µm.

6. A method as claimed in any of claims 1 to 5 in which the diffractive microstructure is a zero order microstructure.

7. A method as claimed in claim 4, wherein the diffractive microstructure comprises a grating, the grating having a period between 0,2µm and 0,6µm.

8. A method as claimed in any preceding claim, wherein the article is a solid pharmaceutical product not in powder form.

9. A method as claimed in any of claims 1 to 8, wherein the article is a foodstuff.

## Patentansprüche

1. Verfahren zum Erzeugen eines Artikels (1), umfassend:
Ausbilden einer lichtbeugenden Mikrostruktur (2, 3) an einer Grenzfläche des Artikels, wobei das Verfahren umfasst:
Ausbilden des Artikels mittels eines Press- oder Formvorgangs, wobei die Mikrostruktur (2, 3) dadurch in dem Press- oder Formvorgang des Artikels ausgebildet wird, dass das Press- oder Formwerkzeug (31, 32) in einer negativen Version der Mikrostruktur ausgebildet ist,
**gekennzeichnet durch**
Beschichten des Artikels mindestens in einem Bereich, der die Mikrostruktur abdeckt, mit einer transparenten Schicht, die einen höheren Brechungsindex als die Mikrostruktur hat.

2. Verfahren gemäß Anspruch 1, wobei der Artikel ein festes pharmazeutisches Produkt ist, das durch Pressen eines Pulvers in eine feste Substanz ausgebildet wird, wobei die Mikrostruktur auf einer Oberfläche der festen Substanz während des Pressschritts durch Vorsehen einer negativen Urform in dem Formpresswerkzeug ausgebildet wird.

3. Verfahren gemäß einem der Ansprüche 1 oder 2, wobei während des Ausbildungsschritts mindestens die Oberfläche des Artikels mindestens lokal geschmolzen wird.

4. Verfahren gemäß einem der Ansprüche 1 bis 3, wobei der Artikel oder seine Oberfläche nicht vollständig transparent oder metallisch reflektierend, sondern absorbierend oder streuend ist.

5. Verfahren gemäß einem der Ansprüche 1 bis 4, wobei die lichtbeugende Mikrostruktur ein Gitter umfasst, wobei das Gitter eine Periode zwischen 0,75 µm und 3 µm hat.

6. Verfahren gemäß einem der Ansprüche 1 bis 5, wobei die lichtbeugende Mikrostruktur eine Mikrostruktur nullter Ordnung ist.

7. Verfahren gemäß Anspruch 4, wobei die lichtbeugende Mikrostruktur ein Gitter umfasst, wobei das Gitter eine Periode zwischen 0,2 µm und 0,6 µm hat.

8. Verfahren gemäß einem der vorhergehenden Ansprüche, wobei der Artikel ein festes pharmazeutisches Produkt ist, das nicht in Pulverform ist.

9. Verfahren gemäß einem der Ansprüche 1 bis 8, wobei der Artikel ein Nahrungsmittel ist.

## Revendications

1. Procédé de production d'un article (1) comprenant la formation d'une microstructure diffractive (2, 3), en une interface de l'article,
dans lequel le procédé comprend la formation de l'article au moyen d'une opération de pressage ou de moulage, la microstructure (2, 3) étant formée au cours du processus de pressage ou de moulage de l'article en formant l'outil de pressage ou de moulage (31, 32) avec une version négative de la microstructure, **caractérisé par** le revêtement de l'article, au moins dans une zone couvrant la microstructure, avec une couche transparente ayant un indice de réfraction plus élevé que la microstructure.

2. Procédé selon la revendication 1 dans lequel l'article est un produit pharmaceutique solide formé en pressant une poudre en une masse solide, la microstructure étant formée sur une surface de la masse solide pendant l'étape de pressage en fournissant une matrice négative dans l'outil de pressage.

3. Procédé selon l'une des revendications 1 ou 2 dans lequel, pendant l'étape de formation, au moins la surface de l'article est fondue au moins localement.

4. Procédé selon l'une des revendications 1 à 3 dans lequel l'article ou sa surface n'est pas complètement transparent ou réfléchissant métallique mais absorbant ou diffusant.

5. Procédé selon l'une des revendications 1 à 4 dans lequel la microstructure diffractive comprend un réseau, le réseau ayant une période comprise entre 0,75 µm et 3 µm.

6. Procédé selon l'une des revendications 1 à 5 dans lequel la microstructure diffractive est une microstructure d'ordre zéro.

7. Procédé selon la revendication 4 dans lequel la microstructure diffractive comprend un réseau, le réseau ayant une période comprise entre 0,2 µm et 0,6 µm.

8. Procédé selon l'une des revendications précédentes, dans lequel l'article est un produit pharmaceutique solide qui n'est pas sous forme de poudre.

9. Procédé selon l'une des revendications 1 à 8, dans lequel l'article est une denrée alimentaire.
